# EUROPEAN PATENT APPLICATION

(11) **EP 3 680 834 A1**
(43) Date of publication of application: **15.07.2020**
(21) Application number: 19208341.8
(22) Date of filing: 20.01.2016
(51) Int. Cl.: G06Q 10/06, G16H 10/40

(54) **PROCESS SIMULATION IN CELL A PROCESSING FACILITY**

(30) Priority: 20.01.2015 US 201562105330 P
(62) Divisional of application: 16702892.7
(71) Applicant: Global Life Sciences Solutions USA LLC, Marlborough, MA 01752 (US)
(72) Inventor: BAKSH, Dolores, Malborough, MA 01752-7217 (US); KUNTER, Akbay, Seref, Niskayuna, NY 12309 (US); WOOD, Nichole, Lea, Niskayuna, NY 12309 (US); SMITH, Reginald, Donovan, Niskayuna, NY 12309 (US)
(74) Representative: Cavill, Ross David

(57) **Abstract**

The present invention provides improved methods, facilities and systems for parallel processing of biological cellular samples in an efficient and scalable manner. The invention enables parallel processing of biological cellular samples, such as patient samples, in a space and time efficient fashion. Process simulation may be used to determine the optimal arrangement and/or quantity of cell processing equipment needed. The methods, facilities and systems of the invention find particular utility in processing patient samples for use in cell therapy.

## Description

### BACKGROUND TO THE INVENTION

Cell therapy is a key area of medical advance in the treatment of a range of conditions and diseases including cancer. Autologous cell therapy, the treatment of a patient with the patient's own cells, is an increasingly used and improving method for combatting cancers, including melanoma and leukaemia, which are refractory to conventional drug treatment. One area of autologous cell therapy, immunotherapy, uses selection and expansion of cells from the patient's own immune system to target and attack cancer cells, effectively boosting, many fold, the patient's immune response to destroy the cancer cells.

To achieve immunotherapy and other forms of cell therapy samples of cells taken from a patient, typically in the form of a blood sample, must be processed through a complex workflow to isolate, engineer, concentrate and/or expand by culture the cells which will form the therapeutic material administered back into the patient. Carrying out the cell processing workflow requires a series of operations performed using a variety of processing methods, machines and instruments, each with a unique role in the overall process. The process may comprise steps of different duration and complexity requiring varying degrees of operator intervention and skill and all operations must be carried out under sterile conditions to prevent microbial, viral or other contamination of the patient sample. The process must also be carried out using means which maintain the integrity of the patient's material and prevent partial or whole cross-contamination or mixing of patient samples to prevent a patient receiving a therapeutic preparation which is not wholly derived from the patient's own cells.

To achieve the sterility and integrity of patient material all processing operations are typically performed in a laboratory or clean room furnished with equipment, for example laminar air flow cabinets, which allow the material to be manipulated using open containers in a sterile environment to minimise the risk of biological or other contamination from the environment. To prevent mixing of patient materials and maintain the integrity of the sample identity the processing operations are carried out in separate and isolated processing rooms or units each of which duplicates the equipment and processes of the others. Each duplicated unit provides the necessary sterile working environment and is furnished with all of the sample handling and processing equipment required to process one single patient sample at one time. As each unit is used only for one patient sample at a time, a facility processing many patient samples requires a number of identical processing units and therefore duplicates costs of providing space, services and equipment, such costs scaling linearly with the number of patient samples to be processed. These costs are seen as a major barrier to the further development of cell therapy and the expansion of use of cell therapy in a larger patient population as the duplicative approach does not provide economies of scale to reduce treatment costs.

In addition to the high setting up and running costs and the high costs of capacity expansion, the duplication of processing units is extremely inefficient in use of space and equipment. Since each stage of the processing workflow takes a different period of time, the overall throughput of the workflow is determined by the rate limiting step, i.e. the longest step in the process, and therefore most of the resources available in each duplicated processing unit are underutilised for much of the time taken to process a sample through the workflow. In a typical immunotherapy processing workflow the process of cell expansion, the culture and growth of cells from the thousands of cells isolated from a patient's blood sample to the millions or billions of cells required for a therapeutic dose, may take up to two weeks. In contrast, the cell isolation and concentration steps used at the beginning and end of the workflow may take only a few minutes or hours. Consequently in the standard cell processing facility, using duplication of processing units, a large amount of space and capital equipment used for short term operations, such as cell isolation, stands idle during the cell expansion operation.

In addition to the cost and efficiency shortcomings of the standard duplicated unit approach described above, processing samples in a laboratory or clean room using open containers still retains a risk of bacterial, viral or other contamination of the sample, does not preclude loss of part or all or the patient sample or processed material at any stage in the process due to operator error, and retains the opportunity for cross-contamination of samples by residual material remaining in the processing unit from a previous patient sample or processed material.

What is required is a means to process patient material in a fashion which maximises the efficiency of the processing workflow for time and cost allowing the process to be operated for multiple patients with economies of scale that enable use of cell therapy in a larger patient population. Such means must retain the fundamental key principles of preventing contamination, mixing, loss of identity or other events which interfere with the physical and identity integrity of the patient sample and processed therapeutic material.

These features and benefits are not provided by current cell therapy processing facilities and such features and benefits are not described or suggested by the prior art.

### SUMMARY OF THE INVENTION

According to a first aspect of the present invention there is provided a method for processing of a plurality of biological cellular samples, implemented using process simulation modelling, and preferably operated using real time process simulation to substantially optimise the production of said cells. The cells produced by said method fall within the ambit of the invention.

### BRIEF DESCRIPTION OF THE DRAWINGS

Figure 1: Schematic of a unitised parallel processing facility illustrating the workflow of patient samples and processed materials through discrete workflow units comprising processing stations and processing components.
Figure 2: Schematic of an identity custody chain for patient sample and processed material illustrating means to achieve physical and identity integrity through the use, tracking and recording of uniquely encoded disposable components.
Figure 3: Schematic of means of maintaining the physical and identity integrity of sample and processed material illustrating means to achieve connection of disposable closed processing components preventing mixing, loss or contamination of sample or processed material through use of encoded connectors.
Figure 4: Schematic of means of providing processing instructions to a processing station from an instruction store.
Figure 5: Schematic of means of providing processing instructions to a processing station from a processing component.
Figure 6: A flow diagram which shows the steps performed for process simulation.
Figures 7&8: Tables of modelled data.
Figure 9: An example of a manufacturing layout.

### DETAILED DESCRIPTION OF THE INVENTION

A scalable cell therapy facility comprises a number of discrete processing units (UNIT 1 to UNIT N) isolated from one another by physical walls, barriers or other demarcation. Each processing unit comprises a number of identical processing stations (P1/1 to P1/n in UNIT 1; P2/1 to P2/n in Unit 2; PN/1 to PN/n in UNIT N) appropriate for the unique processing operation to be carried out within the unit. Patient samples (S1 to Sn) are received by UNIT 1 in uniquely encoded closed sample containers and processed on processing stations P1/1 to P1/n using a separate uniquely coded closed disposable processing component 1 for each sample. Processed samples in closed components appropriate to the workflow stage are sequentially passed through UNIT2 to UNIT N to complete the processing workflow using uniquely coded closed processing components 2 to N at each stage. At each stage of processing transfer of processed patient material from component to component is tracked by recording component unique identities maintaining an identity custody chain.

UNIT 1 to UNIT N may comprise physically separated rooms or zones within a facility with the operations of processing platforms and handling and transfer of components and samples being carried out by one or more operating staff. Alternatively UNIT1 to UNIT N may comprise designated areas within a larger area or room where processing platforms operate automatically and transfer of components and samples is performed by one or more robot devices. The facility comprising UNIT 1 to UNIT N may be housed within a larger facility, such as a hospital or other treatment centre, or may be a self-contained unit capable of independent operation. The facility may be housed in a prefabricated building, vehicle, craft, vessel or other container suitable for deployment to a suitable location for processing cell therapy materials. The facility may be situated locally or remotely to patients providing samples and/or undergoing treatment. Where the facility is located remotely to patient sampling and/or patient treatment locations patient samples and/or final therapeutic materials are transported from and/or to patients in sealed uniquely encoded containers and remote location(s) are connected to the facility by means to allow transmission and receipt of patient and sample identities to provide means to maintain physical and identity integrity for samples and processed materials.

The parallel processing facility maintains physical separation of samples within the processing units by use of disposable closed processing components at all stages in the processing work flow from sample receipt to formulation of the therapeutic material for administration. The facility is readily scalable by increasing the number of processing stations in each unit and the numbers of processing stations in each unit may be tailored to provide the optimum efficiency and throughput to the facility by having a larger number of stations in units where the processing step has a long duration and a smaller number of stations in units which short processing steps (e.g. a small number of stations in the sample isolation unit; a larger number of stations in the cell expansion unit). Segregation of processing stations by function enables the provision of the optimum environment (lighting, electrical power and other services, temperature control etc.) required for the processing stations within a common unit. These characteristics of the unitised parallel processing facility provide a number of key advantages over the shortcomings of conventional duplicated parallel operations where all processes for a single patient are carried out within a separate room (e.g. redundant duplication of equipment, scalability requiring additional space and equipment services).

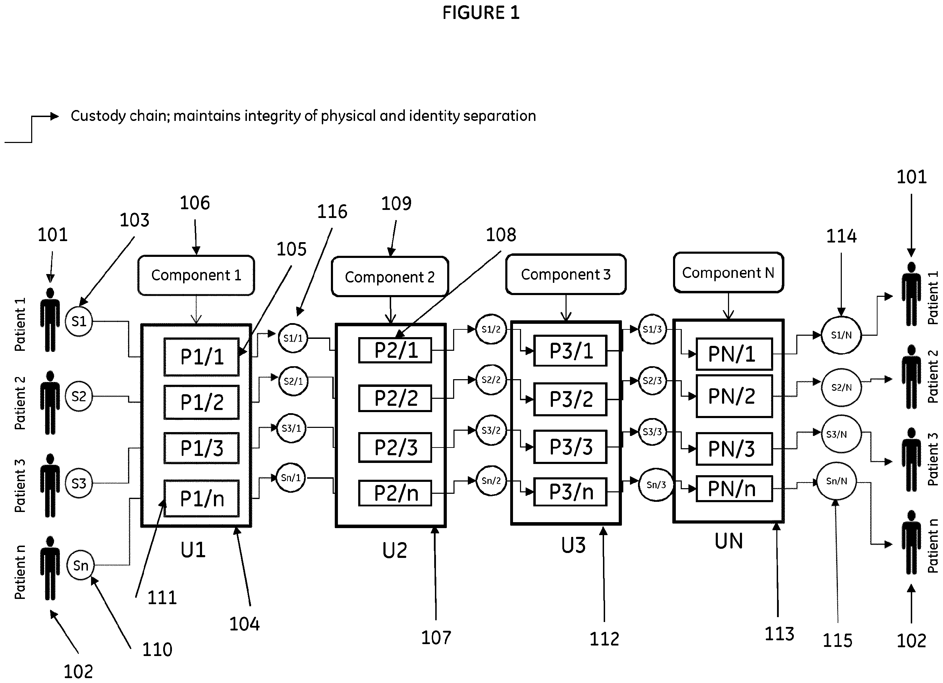

Description of one possible illustrative embodiment of the scalable cell therapy processing facility is made with reference to Figure 1. The facility comprises a number of processing cells (UNIT 1 to UNIT N) wherein samples from Patient 1 [101] to Patient n [102] are processed in parallel in separate closed disposable containers within the facility to maintain patient sample integrity and identity at all times. A sample S1 [103] containing cells from Patient 1 [101] is collected in a uniquely encoded disposable container and transferred to UNIT 1 [104] to begin processing. UNIT 1 [104] comprises a number of processing stations P1/1 [105] to P1/n [111] suitable for performing the first step in the cell processing work flow. Patient sample S1 [103] is processed on processing station P1/1 [105] using a uniquely encoded disposable processing component 1 [106]. Other samples from Patient 2 to Patient n [102] are processed in parallel with sample n [110] from Patient n [102] processed on processing station P1/n [111] using a uniquely encoded disposable processing component 1 [106]. Following completion of processing in UNIT 1, sample 1 [116] is moved in a closed container to the next processing unit, UNIT 2 [107] for the next stage of processing on processing station P2/1 [108] using a uniquely encoded disposable processing component 2 [109] suitable for the processing operation to be carried out. Processing of samples continues in parallel through processing units UNIT 3 [112] to UNIT N [113] in which the final stage of processing is performed using a separate uniquely encoded disposable processing component for each processing stage and each patient sample. The fully processed therapy sample 1 [114] is transported in a uniquely encoded disposable closed container for administration to Patient 1 [101] from whom the starting sample [103] was taken. Other samples from Patient 2 to Patient n are similarly processed in parallel through the facility at all times being isolated in enclosed uniquely encoded disposable containers with the fully processed therapy sample n [115] being administered to Patient n [102] from whom the starting sample [110] was taken.

The preceding description of one possible embodiment of the present invention is provided for illustrative purposes only. Those skilled in the art will readily appreciate that other means of providing the key required features of the present invention for a unitised parallel processing cell therapy facility are possible.

All components in the processing chain, including an identity bracelet or other identification means worn by the patient, carry unique encoding. Suitable encoding means include but are not limited to encoding using tags in printed, magnetic or electronic form which may be read by light, electronic or magnetic means, such as barcodes, QR codes, RFIDs or transponders. It will be readily understood by those skilled in the art that a variety of encoding means are suitable for use in the method of the current invention. One suitable encoding means comprises light activated micro-transponders, such as those from the PharmaSeq company described in WO2002037721, US5981166 and US6361950, which are small (500 x 500 x 200µm) low cost silicon devices which store a unique 30 bit read-only identity code and emit the code as radio frequency signal when powered and interrogated with a light emitting reader device. All processing components (sample collection tube, cell purification components, cell culture and expansion components etc.) are pre-registered in a facility component registry where each component's function and intended stage of use in the processing workflow is logged against the component's unique identifier code. In the descriptions of embodiments described herein the term 'transponder' is intended to encompass any means of encoding a unique sample identity which may be read by suitable reading means.

At each stage in the therapy processing workflow the identifier code is read into a unique patient specific record in a central database. The first entry in the database is the identity code from the patient bracelet. At sample collection (e.g. blood collection) the sample collection component identity code is read and two actions are carried out;
1. The sample collection component identity code is checked against the component registry to confirm the correct component is being used for that stage in processing and;
2. The sample collection component identity code is added as the second entry to the custody chain of component identity codes in the patient record.

Following sample collection the filled collection component is transferred to the next operation in the processing workflow to perform a processing step using a processing component specific to that workflow stage and two actions are carried out;
1. The processing component identity code is checked against the component registry to confirm the correct component is being used for that stage in processing and;
2. The processing component identity code is added as the third entry to the custody chain of component identity codes in the patient record.

Processing of the patient sample continues through the necessary operations with each transfer of physical sample from component to component being accompanied by the check and record actions 1 & 2 with the processing components being added as the fourth to the nth entry in the custody chain.

At the end of the processing workflow when the therapeutic material is ready for administration to the patient the following actions are carried out;
1. The identity codes of the component containing the therapeutic material and the patient identity bracelet are both read and;
2. The patient record data base custody chain of component identity codes is checked stepwise to ensure that all component identity codes track back to the same patient identity.

Further features of the custody chain include the ability to link all component identity codes to electronic manufacturer's and/or supplier's batch records whereby scanning of the component appends electronic copies of component batch record files to the patient record file to enable traceability of all components used in processing the patient's sample. In addition all commercially supplied reagents (e.g. cell growth media) carry transponders on their containers with identity codes linked to the manufacture's batch records allowing electronic copies of records, certificates of analysis etc. to be appended to the patient record. To allow for the use of non-commercially supplied, bespoke or other special reagents or formulations which may be prepared within the facility, additional encoded reagent containers are provided for filling and storage of facility produced reagents (e.g. virus preparations for transduction of CAR T-cells in cancer immunotherapy).

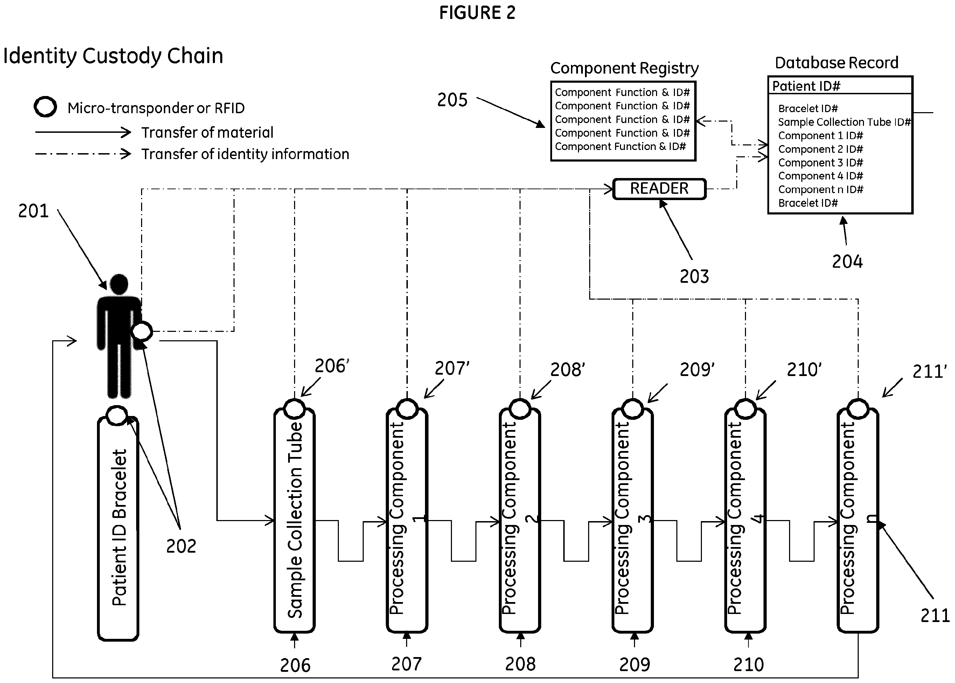

These principles are demonstrated in the following illustrative embodiment by reference to Figure 2. The patient undergoing cell therapy wears an identity bracelet [201] or other non-removable identifying device comprising a unique readable transponder code [202]. The transponder code is read by a reader [203] connected to a central database and the code stored in the patient's individual database record [204]. At the first stage in the cell therapy process a sample, for example of blood, is taken from the patient into a sample collection tube or container [206] carrying a unique transponder code. The transponder code for the sample collection tube or container is read by the reader [203] and the identity code for the filled tube or container stored in the patient's database record [204]. The transponder code is also used to check the component function by reading a component registry [205] containing component functions matched to component transponder numbers for all components in the cell processing workflow. To further process the sample collection tube or container containing the patient's blood sample the sample collection container or tube [206] must be connected to the first component [207] in the processing workflow. Prior to connection the transponder on the first component [207] is read by the reader [203] and checked against the component registry [205] to confirm if the component is the next correct component in the processing sequence. If the component is correct the component transponder code is appended to the patient's database record [204]. If the component is not correct the operator is notified to select the correct component. The sample is sequentially processed through each stage in the workflow using processing components 2 [208], 3 [209], 4 [210] through to processing component n [211] with the number of components determined by the complexity and steps in the workflow. At each stage in sample transfer between components the transponder codes on each component are read by the reader [203], checked against the component registry [205] and recorded in the patient's database record [204]. When sample processing is complete and the therapeutic material is present in the last processing component [211] ready for administration to the patient the transponder code on the component [211] and on the patient identity bracelet [202] are read on the reader [203] and the identity numbers checked against the patient record in the database record [204] to ensure that the transponder identity number for the final component containing the therapeutic material [211] tracks back through the custody chain of successive transponder codes stored in the database record [204] to the same patient identity bracelet [202] transponder code read at sample collection. Matching of all transponder component identity codes in the patient database record [204] confirms that the sample and therapy relate to the same patient in the identity custody chain and therapy can proceed by administration of the sample stored in the final processing container [211].

The described embodiment is provided for illustrative purposes only and those skilled in the art will appreciate that other means of achieving an identity custody chain providing the key features of the invention are possible.

A further key aspect of the present invention is means to achieve a physical and identity custody chain which prevents contamination, cross-contamination or partial or whole loss of a patient sample by environmental exposure in a non-sterile environment or through operator error. All samples and processed materials are handled, processed and stored in closed disposable containers which are specific to each stage of the processing workflow and interface with each processing station in the workflow. All such process components are joined by connection means which prevent;
1. Cross contamination of patient samples by cross-mixing of parallel processing sample workflows being performed in the same processing unit.
2. Loss of patient sample or processed material through the incorrect order of use of components.

To maintain the physical separation and identity of the processed patient sample all connections between processing components 1 to N in the processing workflow are made using connectors furnished with means to prevent loss, mixing or cross-contamination of the sample integrity through operator error. Such connectors are designed and operated to;
A. Allow only the correct sequence of processing components to be used in processing the patient sample preventing loss of the patient sample through use of incorrect components in sequential steps of the processing workflow.
B. Allow only components linked to the patient identity to be coupled together preventing mixing or cross-contamination of the sample with another sample being processed through the facility in parallel.
C. Maintain a record of the identity of the patient sample at all stages in the workflow preventing mixing or cross-contamination of the sample with another sample being processed through the facility in parallel.
D. Prevent the re-use of components preventing mixing or cross-contamination of the sample with another sample being processed through the facility in parallel.

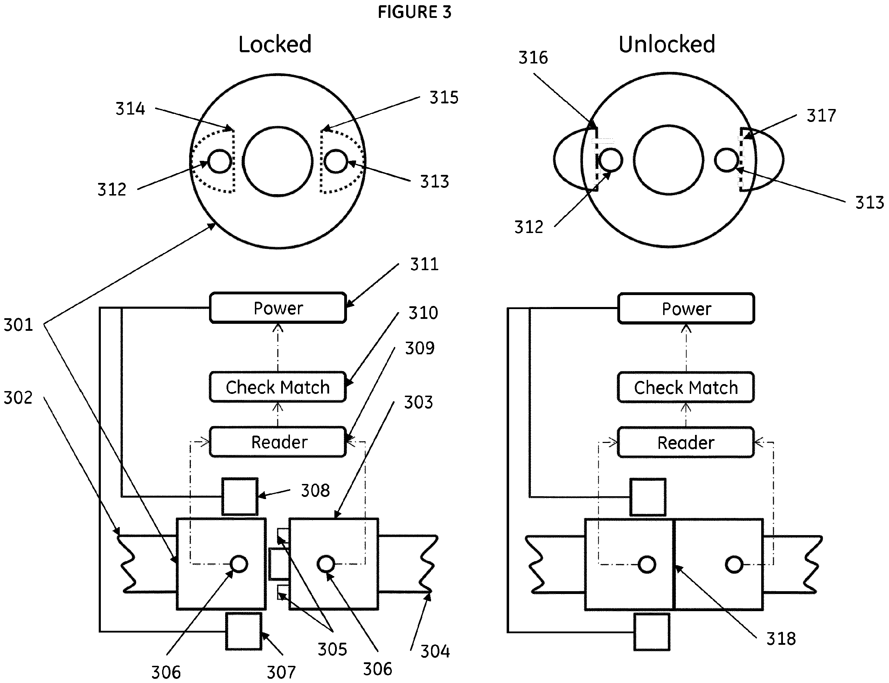

These principles are demonstrated in the following illustrative embodiment by reference to figure 3. Connectors providing sample physical and identity integrity comprise a female [301] connector linked via tubing [302] to a first processing component and a male connector [303] linked via tubing [304] to a second processing component. The male connector [303] and the female connector [301] are designed so as to form a liquid- and air-tight junction between two components when correctly connected. The connectors are further provided with means to establish a sterile connection when connectors are joined together in a non-sterile environment, such as that described in US 6679529. The male connector [303] carries blocking pins [305] orientated to fit into location holes [312 & 313] located in the front face of the female connector [301]. The blocking pins are prevented from entering the location holes [312 & 313] by metal blocking shields [314 & 315] held in slots within the female connector [301] which prevent coupling of the connectors to form a junction between the processing components. The male and female connectors carry identity transponders [306] encoding the individual identities of the processing components attached to each of the connectors. To form a join between the connectors the male [303] and female [301] connectors are placed in a reading device [309] comprising means to align the connectors and means to read information from the identity transponders [306] carried on each connector. On activation of the reader [309] the identity codes of the two connectors are read and the device software performs a component compatibility match check [310] to determine whether the two connectors present in the device form a correct sequential component coupling for sample processing. Additional checking is performed by the reader [309] software to further ensure the physical separation and identity of the patient sample, for example the identity codes from the transponders [306] are checked to ensure that the component being offered to receive the patient sample at a step in the processing workflow is not a waste component having been previously used. If the match checking operation [310] confirms the correct identity of the paired connectors a power supply [311] is activated to energise electromagnets [307 & 308] held within the reading device. Activation of the electro magnets pulls the blocking shields [314 & 315] outwards and away from the location holes [312 & 313] in the female connector to an open position [316 & 317] allowing the blocking pins [305] in the male connector to enter the location holes [312 & 313] in the female connector. The connectors are now pushed together to provide a secure operating connection [318] between the processing components. Following correct connection the reader [309] additionally records the identity code of each connector from the transponders [306] and sends the data to the patient sample record to provide a sample identity custody chain. If the match checking operation [310] detects that the two connectors do not have the correct identities to form a correct sequential component coupling for sample processing, power is not supplied to the electromagnets [307 & 308] preventing the coupling of the connectors. The reader software then prompts the operator to select the correct components to form an operable connection.

The described embodiment is provided for illustrative purposes only and those skilled in the art will appreciate that other means of providing component connection meeting the required principles of maintaining sample physical and identity integrity may be used. Such means include but are not limited to alternative methods of component encoding such as barcoding, and magnetic strip and RFID tagging to identify correct components for connection. Alternative means for prevention of connection of incorrect sequential components include but are not limited to providing a sequential series of unique connectors with varying mirrored dispositions of pins and holes or grooves and ridges which physically preclude the connection of mismatched connectors. Such connection means can be designed and disposed to ensure that the output from a first component will connect only to the input of a second component, the output from the second component will connect only to the input of a third component and so on for a series of N components with the output of the N-1th component connecting only to the input of the Nth component in the series. Additionally the connectors may be colour and or shape coded to aid in manual or automated selection of correct components and connection pairings.

A further key aspect of the invention is the provision of processing instructions to a processing station directly from, or in response to, a processing component connected to a processing station. Each processing component comprises means to instruct a processing station on the type of processing component and if applicable, the variant type of the processing component and to instruct a processing station on processing the patient sample held within the processing component. A processing component variant type may comprise a different size, capacity or other feature of the component which requires individual processing instructions specific to that variant. Such individual processing instructions may have variant specific instructions for reagent volumes, pressures, flow rates, incubation times etc. which are specific for the optimum operation of that processing component variant. For example a processing component for performing cell isolation may be provided in two variants for processing different volumes of blood; such variants will require different reagent volumes and hence different processing instructions. Similarly a processing component used for cell expansion, such as a disposable bioreactor for cell culture, may be provided in different sizes and culture capacities to allow the growth of different numbers of cells for use in therapy; such variants will utilise different volumes of culture media and different processing instructions.

Linking processing instructions to a processing component and providing such instructions to a processing platform operably connected to the processing component provides;
a) means to ensure that the instructions for processing a patient sample within the processing component are correct for that component, obviating risk of sample loss through use of incorrect processing instructions.
b) means to ensure that variants of processing components performing the same operation at a different scale are provided with specific processing instructions necessary for the correct processing.
c) means to remove operator errors by directly instructing processing stations.
d) means to permit processing to be carried out in an automated environment using robotic means to achieve the processing workflow where each processing station in the workflow is appropriately instructed to perform a processing operation on receipt of a processing component.

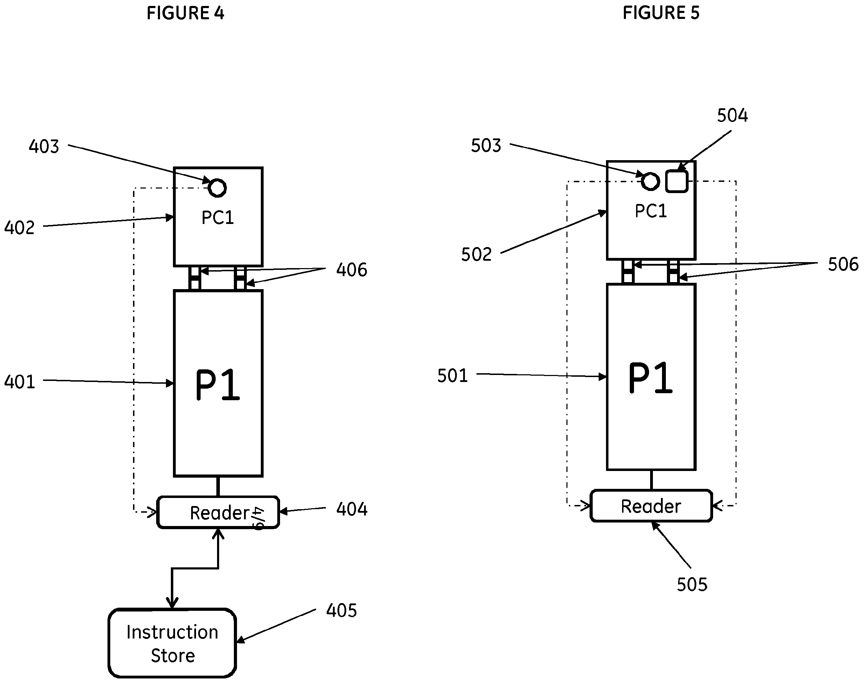

These principles are demonstrated in the following illustrative embodiments by reference to figure 4. In a first further embodiment of the invention a processing component [402] is operably connected to a processing station [401] by connectors [406] to permit sample processing wherein the processing component comprises a transponder [403] carrying a unique identity code. The unique identity code is linked to a database in a central instruction store [405] to specific processing instructions for the type and variant of processing component carrying the transponder [403]. The identity code carried by the transponder [403] is read by a reader [404] connected to the processing station [401] and checked to confirm that the processing component is of the correct type for processing on the processing station [401]. On receipt of the identity code the reader [404] retrieves processing instructions from the instruction store [405] by wired or wireless communication and the received instructions are passed to the processing station [401] to permit the correct operation of the processing station in processing the patient sample contained in the processing component [402].

In a second further embodiment of the invention (Figure 5) a processing component [502] is operably connected to a processing station [501] by connectors [505] to permit sample processing wherein the processing component comprises a transponder [503] carrying a unique identity code. The processing component [502] additionally comprises a stored processing instruction set [504] specific to the type and variant of the processing component [502]. The identity code carried by the transponder [503] is read by a reader [505] connected to the processing station [501] and checked to confirm that the processing component is of the correct type for processing on the processing station [501]. The processing instruction set [504] is also read by the reader [505] by wired or wireless means and the processing instructions passed to the processing station [501]. The processing instruction set [504] carried by the component [502] may be stored and read by a variety of means including, but not limited to, storage of processing instructions by barcoding, QR coding, magnetic and solid state memory, and reading of processing instructions by optical or electronic means. In a further variant identity coding and instruction storage may comprise a single data store carried on each processing component.

In a further embodiment analytical means are used to ensure matching of a patient sample and a therapeutic material derived from the sample to ensure identity integrity is maintained through processing. The patient sample is subjected to a suitable chemical, biochemical or molecular analysis and a first biomarker signature characteristic of the sample is stored on the patient's database record. Following processing of the sample the resulting therapeutic material is analysed using the same analytical method and a second biomarker signature is stored on the patient's database record. Prior to administration of the therapeutic material the first biomarker signature of the original patient sample and the second signature of the therapeutic material are checked to verify a match between the two signatures confirming that the patient sample and the processed material are both derived from the same patient.

Suitable analytical means include, but are not limited to, analysis of proteins, RNA and DNA. Suitable means for deriving a signature of protein biomarkers include analysis of cellular proteins, including but not limited to, HLA antigens and blood group proteins by flow cytometry, ELISA or western blotting. Suitable means for deriving a signature for RNA and/or DNA include, but are not limited to, PCR, RT-PCR, DNA sequencing, SNP analysis, RFLP analysis, genetic fingerprinting and DNA profiling. Particularly suitable methods include those in standard use in forensic medicine which analyse DNA repeat sequences that are highly variable such as variable number tandem repeats (VNTR) and in particular short tandem repeats (STR) which are so variable that unrelated individuals are extremely unlikely to have the same VNTR. Such means can be used to unambiguously assign a patient identity to a processed therapeutic material by matching the STR signature of the original patient sample and the therapeutic material.

Whilst the above description describes ways in which parallel processing of cellular material can be performed efficiently and in safety, the need for optimisation of the cell culture procedure needs to be addressed also. Process simulation is a known process but is applied to cell culture in a novel way herein, to provide not only optimisation of the parallel process described above, but also of any large scale (50 or more doses annually) cell culture process for therapeutic applications. Herein in an embodiment, process simulation techniques are used whereby a software model is provided of the process units U1,U2, U3 UN as shown in Figure 1, including their operation capacity, inputs needed and to iteratively determine the optimal operating conditions for various output demands (i.e. therapeutic cell doses required per annum). This is done by interpolation and/or extrapolation of the known operating parameters. This process simulation is a useful tool for strategic planning. Presently cellular culturing processes require re-purposed bioprocessing tools, which were not designed for large scale cell culture, for example of autologous cell batches. There is poor physical and data interconnectivity between such equipment, which leads to regulatory concerns. Using simulation modelling it is possible also to design a cell culturing facility that makes optimal use of the individual components of the system employed.

The advantages of process simulation are that it:
provides predictive and actionable feedback on "as is" process when used in real time;
provides a substantially optimal answer regarding required resource levels, scheduling, etc.;
identifies new constraints (including non-obvious ones);
enables the exploration of the merits of alternative facility layouts; and
provides unique "facility designs ", developed to meet larger scale operations, which can be of compact footprint, automated, and have combined processes.

Process simulation can be used prior to operations, to:
Model real-time operations;
Predict future bottlenecks and mitigation strategy;
Combined with mass data analysis to perform pattern recognition and to plan facilities or to modify existing facilities.
Overall process simulation can deliver a robust cell culturing facility which manufactures patient samples consistently, to ensure a therapeutic dose of cells is delivered in a timely manner.

It has been recognised by the inventors that the simulation process of the present invention needs to determine:
1) What are the required resources in order to process 'n' patient samples per year?
2) What resources are required if the annual volume is increased given a set of constraints?
3) How do the different resource levels impact annual throughput and average cycle time?

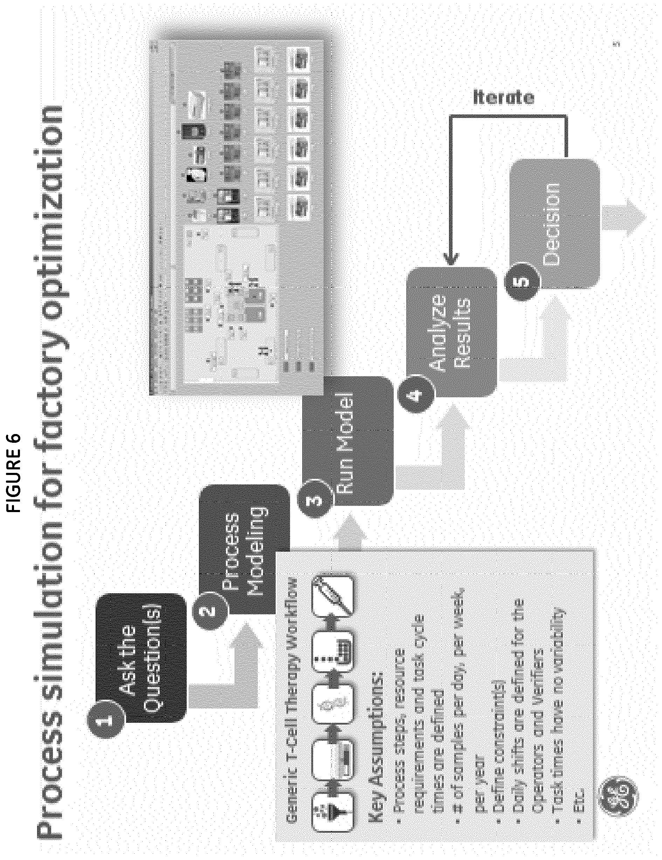

Figure 6 shows a flow diagram which shows the steps performed for process simulation where the above questions are input (box 1), modelled (box 2), simulated in the model (box 3), analysed (box 4) and a decision made (box 5). Into the model, additional inputs are made in the form of data relating to: what process steps are performed- these process steps, for example are those described in relation to Figures 1 and 2; what resources will be required for those steps- for example operators, incubators, cell culture bags, growth media requirements and consumable items, including processing durations; number of input samples per unit time; constraints- for example the need to wait for resources to become available, or limits to cell growth rates; operator working hours and so on.

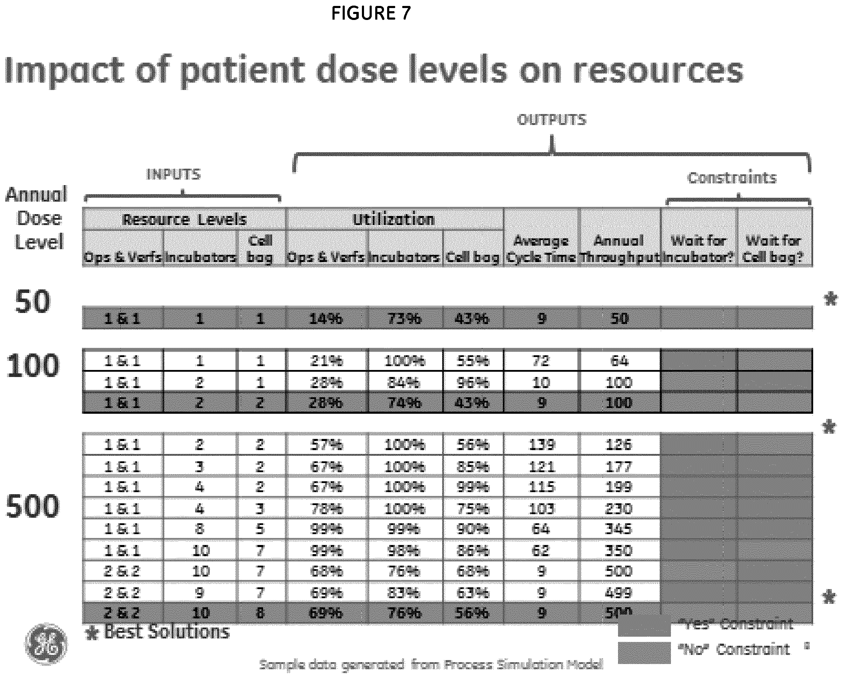

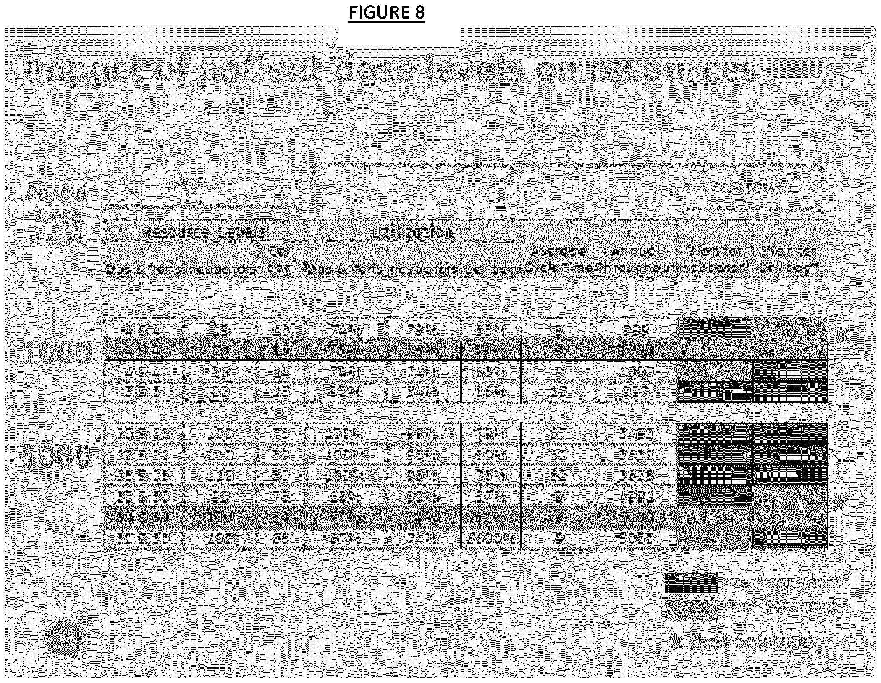

Figures 7 and 8 show the data relating to the above process simulation where 'Ops & Verfs' refers to 'operators and verifier personnel numbers', and 'Incubators' and 'Cell bags' refer to cell culture equipment examples. Using process simulation modelling, the mix of resources for various therapeutic dose quantities per annum has been modelled and the optimum resources have been found, the best being shown with an asterisk.

From the data above an optimum manufacturing layout can be better determined.

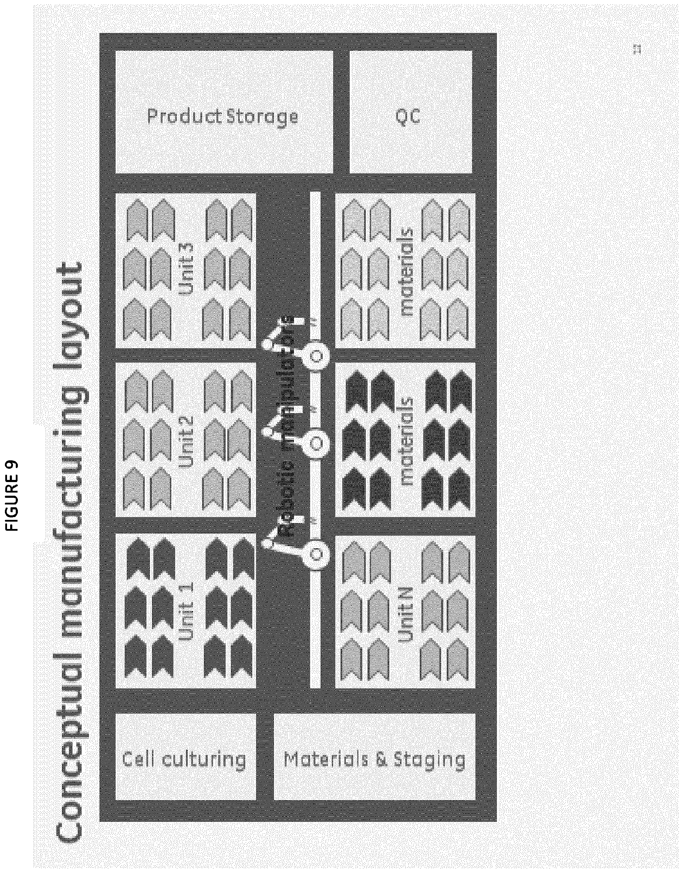

Figure 9 shows that each suite can be optimized for size and capacity, to run one particular process step, for example the processing steps described above in relation to units U1, etc shown in Figure 1. Specific capacity constraints can be addressed, thus directly optimizing utilization of the units.

Each cell processing unit (Unit1,2 etc) can be an individual clean room or a bank of cell culturing units if the Units are enclosed. Specific capacity constraints can be addressed which can directly optimise utilisation of different Units. For example if Unit 1 is a cell modification unit, using process simulation modelling , it was found that a further two units, Units 2 and 3, were needed to optimise the culturing process because cell culturing takes longer to perform than cell modification. In the example shown batches of cells are processed in parallel.

In addition the process simulation modelling can be used in real time once the system is running with current and historic data input, to provide predictive analytics, which can then provide early warnings of potential problems, and operator directions if needed. Process modifications can also be incorporated to avoid potential problems.

While preferred illustrative embodiments of the present invention are described, one skilled in the art will appreciate that the present invention can be practiced by other than the described embodiments, which are presented for purposes of illustration only and not by way of limitation. The present invention is limited only by the claims that follow.

### Embodiments provide

**1.** A method for optimising the performance of a cell processing facility, said facility comprising plural cell processing units, and said facility providing processed cell samples for therapeutic use derived from cellular samples, the method comprising the steps of performing process simulation modelling on said cell processing facility to determining, at least, the optimal arrangement and/or number of processing units.
**2.** The method of claim 1, wherein each processing unit includes plural processing stations, and said modelling is used to optimise the number of processing stations at each unit for said optimal performance.
**3.** The method of claim 1 or 2, further comprising performing further process simulation modelling to determine the optimal operating staff numbers, and/or cell process equipment required.
**4.** The method of claim 1, 2 or 3 further comprising performing further process simulation during operation of said facility, including analysis of current operating data and historic operating data in order to predict problematic operating conditions.
**5.** The method of any one of claims 1 to 4 wherein said cell processing facility is used to process 50 or more, preferably 100 or more, more preferably 500 or more, more preferably 1000 or more, more preferably 5000 or more cellular samples per annum to provide said processed cell samples.
**6.** The method of any one of the preceding claims wherein, said process modelling includes the step of determining the optimum ratio of operators and cell processing stations for a predetermined cell dose output.
**7.** The method of any one of the preceding claims 2 to 6 wherein said stations comprise at least cell isolation and concentration equipment and cell incubators, each employing uniquely identified cell containers or bags.
**8.** A cell processing facility including: plural cell processing units operable to perform process steps on cellular samples derived from different patients in separate closed containers each having unique identification; a reader for reading and recording said identification at said units; wherein each unit includes one or plural processing stations which, if plural, are identical or similar within each unit; and wherein the or each unit is:
   1) provided with a quantity of processing stations, the quantity being determined according to predetermined software steps to optimise the ratio of processing stations in each unit based on the desired output of the facility; and/or
   2) operable according to predetermined software steps to optimise the use of the processing stations based on the desired output, and based on additional operating variables.
**9.** Cells produced according to the methods defined in any one of claims 1 to 7, or produced by the cell processing facility of claim 8, optionally, for use in autologous cell therapy.

## Claims

1. A method for processing a patient sample, comprising the following steps, in any suitable order:
a) an identifier code unique to a patient is recorded as a unique identifier in a database patient record;
b) a patient sample is collected into a patient sample collection component and a unique sample collection component identity code is read and recorded in said patient record;
c) the sample collection component identity code is checked against a component registry to confirm the correct component is being used for that stage in processing;
d) the sample collection component identity code is added as an entry to a custody chain of component identity codes in the patient record;
e) following sample collection, the collection component containing the patient sample is transferred to the next operation in the processing where a processing step is performed using a processing component specific to that stage of the processing;
f) thereat, the processing component identity code of the specific processing component is checked against the component registry to confirm the correct processing component is being used for that stage in processing; and
g) the specific processing component identity code is added as an entry to the custody chain of component identity codes in the patient record.

2. A method for processing a patient sample as claimed in claim 1, wherein steps e) to g) are repeated until patient sample processing is complete.

3. A method for processing a patient sample as claimed in claim 1 or 2, wherein further patient processing steps are preformed and, with each transfer of patient sample from one processing component to the next processing component, additional component checks are performed.

4. A method for processing a patient sample as claimed in claim 2 or 3 culminating in the steps of:
a) reading the identity code of the final sample containing component and the patient identifier code and;
b) checking stepwise that the patient record data base custody chain of component identity codes to ensure that all component identity codes track back to the same patient identity.

5. A method for processing a patient sample as claimed in any one of the preceding claims, wherein the custody chain includes batch record or records of one or more manufacturer or supplier whereby scanning of the component appends electronic copies of component batch record files to the patient record to enable traceability of all components used in processing the patient's sample.

6. A method for processing a patient sample as claimed in 5, wherein said batch record(s) is/are appended to said patient record by means of identifying transponders on containers with transponder identity codes linked to the batch record(s) thereby allowing electronic copies of records, or certificates of analysis to be appended to the patient record.
